# EUROPEAN PATENT APPLICATION

(11) **EP 1 972 334 A1**
(43) Date of publication of application: **24.09.2008**
(21) Application number: 07116727.4
(22) Date of filing: 19.09.2007
(51) Int. Cl.: A61K 9/107, A61K 31/5575

(54) **Emulsion Composition Comprising Prostaglandin E1**

(30) Priority: 21.03.2007 US 896268 P; 15.08.2007 US 839055
(71) Applicant: Taiwan Liposome Co., Ltd., Taipei 115 (TW); TLC Biopharmaceuticals, Inc., South San Francisco, CA 94080 (US)
(72) Inventor: Hong, Keelung, South San Francisco, 94080 (US); Kan, Pei, Hsinchu City 300 (TW); Lin, Yi-Fong, Yonghe City (TW)
(74) Representative: Viering, Jentschura & Partner

(57) **Abstract**

An emulsion composition includes prostaglandin E₁ (PGE₁), a phospholipid with a high purity and a non-proton-providing surfactant that improves stability of PGE₁. Embodiments of the emulsion composition include an effective amount of PGE₁, about 1 % to about 30% (w/w) of a pharmaceutically acceptable oil as an oil base based on the weight of the emulsion composition, about 1% to about 30% (w/w) of a phospholipid with a high purity based on the weight of the oil base, about 1.6% to about 40% (w/w) of a non-proton-providing surfactant based on the weight of the oil base, and the balance of the emulsion composition being water.

## Description

### BACKGROUND OF THE INVENTION

The present invention relates generally to an emulsion composition, and more particularly to an emulsion composition comprising prostaglandin E₁ (PGE₁).

Prostaglandins are hormone-like substances that participate in a wide range of body functions such as the contraction and relaxation of smooth muscle, the dilation and constriction of blood vessels, control of blood pressure, inhibition of platelet aggregation and modulation of inflammation. Therefore, prostaglandins have been developed as pharmaceuticals or therapeutic compounds in the treatment of hypertension, thrombosis, asthma, and gastric and intestinal ulcers, for induction of labor and abortion in pregnant mammals, and for prophylaxis of arteriosclerosis.

There have been many approaches to improve the stability or other properties in association with therapeutic functions of prostaglandin emulsions. Japanese Patent Publication No. JP59141518 disclosed a prostaglandin fat emulsion capable of prolonging the *in vivo* half life of prostaglandin A₁ (i.e. PGA₁). U.S. Patent No. 4,684,633 disclosed that when prostaglandins are incorporated into an oil-in-water emulsion, these compounds are not only stabilized but also made adaptable to intravenous administration and that the use of a phospholipid, which is free of phosphatidylethanolamine, as the emulsifier contributes further to the stability of prostaglandins. The patent recites a prostaglandin emulsion composition that further contains a non-ionic surfactant selected from the group consisting of polyalkylene glycols, polyoxyalkylene copolymers, hydrogenated castor oil-polyoxyalkylene derivatives and castor oil-polyoxyalkylene derivatives, gelatin and hydroxyethyl-starch in a proportion of 0.1 to 5 weight parts relative to 1 weight part of prostaglandin in the composition. However, none of the examples provided in this US patent used non-ionic surfactants as emulsifiers. Japanese Patent Publication No. JP4069340 disclosed a stabilized and clear PGE₁ oil base emulsion using high-purity sesame oil instead of soybean oil. Japanese Patent Publication No. JP433833 disclosed a PGE₁ fat emulsion comprising sesame oil as an oil base and a phospholipid as an emulsifier, which is free from higher fatty acid. Japanese Patent Publication No. JP11279082 disclosed a fat emulsion for injection comprising polyethylene glycol-bound phospholipid, lecithin and a fatty oil to provide an improved circulation time of PGE₁ in blood. According to the embodiment, lecithin that is a phosphatidylcholine with a purity of 70% was used to provide the improved circulation time; however, a dipalmitoyl phosphatidylcholine (DPPC) with a higher purity-could not provide such improvement. There is still a need to provide a PGE₁ emulsion composition having a good shelf stability for a relatively long time.

### BRIEF SUMMARY OF THE INVENTION

The present invention provides a PGE₁ emulsion composition with an improved shelf stability. One aspect of the invention relates to an emulsion composition including PGE₁ with an extended shelf stability, wherein the composition comprises a non-proton-providing surfactant as an auxiliary emulsifier.

According to an embodiment of the invention, the emulsion composition comprises an effective amount of PGE₁, about 1 to about 30% percent weight by weight (% (w/w)) of a pharmaceutically acceptable oil based on the weight of the emulsion composition, about 1% to about 30% (w/w) of a phospholipid with a high purity based on the weight of the oil base, about 1.6% to about 40% (w/w) of a non-proton-providing surfactant based on the weight of the oil base, and the balance of the composition being water.

Another general aspect of the present invention relates to a method of improving the stability of PGE₁ in an emulsion composition, comprising mixing together an effective amount of PGE₁, a pharmaceutically acceptable oil, an emulsifier, and about 1.6% to about 40% (w/w) of a non-proton-providing surfactant based on the weight of the pharmaceutically acceptable oil.

A further general aspect of the present invention relates to a method of treating a disease or disorder related to PGE₁ in a subject, comprising administering to the subject a therapeutically effective amount of an emulsion composition according to any of the embodiments of this invention.

Additional aspects and advantages of the invention will be set forth in part in the description which follows, and in part will be apparent from the description, or can be learned by practice of the invention. The aspects and advantages of the invention will be realized and attained by means of the elements and combinations particularly pointed out in the appended claims.

It is to be understood that both the foregoing general description and the following detailed description are exemplary and explanatory only and are not restrictive of the invention, as claimed.

### DETAILED DESCRIPTION OF THE INVENTION

Various publications, articles and patents are cited or described in the background and throughout the specification; each of these references is herein incorporated by reference in its entirety. Discussion of documents, acts, materials, devices, articles or the like which has been included in the present specification is for the purpose of providing context for the present invention. Such discussion is not an admission that any or all of these matters form part of the prior art with respect to any inventions disclosed or claimed.

Unless defined otherwise, all technical and scientific terms used herein have the same meaning as commonly understood to one of ordinary skill in the art to which this invention pertains. In this application, certain terms are used frequently, which shall have the meanings as set in the specification. It must be noted that as used herein and in the appended claims, the singular forms "a," "an," and "the" include plural referents unless the context clearly dictates otherwise.

One general aspect of the present invention is directed to an emulsion composition comprising a PGE₁ with an extended shelf stability. The emulsion composition specifically includes a non-proton-providing surfactant as an auxiliary emulsifier. According to an embodiment of the invention, one or more non-proton providing surfactants are used to stabilize the oil droplets and maintain the size distribution of the oil droplets. Accordingly, the present invention relates to an emulsion composition comprising PGE₁ with a relatively high shelf stability compared to the prior art, which can provide a substantially large amount of the PGE₁ remaining or retained in the emulsified composition or emulsion without degradation during an extended period of storage, such as for one week or more than one month.

The term "shelf stability" as used herein, refers to the tendency of PGE₁ to remain in substantially the same physical form, and in a relatively high amount, which is substantially more than 80% of the PGE₁ amount originally contained in the emulsified composition. In particular embodiments of the invention, the shelf life of the emulsified composition stored at about 4°C is more than about 24 months and that at about 10°C is more than about 15 months, as compared with those of a conventional composition being only about 15 months, and about 9 months, respectively. Further, according to an embodiment of the invention, the shelf life of the emulsified composition stored at about 40°C is more than one week.

In accordance with one example of the invention, the emulsion composition provides an effective amount of PGE₁, about 1% to about 30% (w/w) of a pharmaceutically acceptable oil as an oil base based on the weight of the emulsion composition, about 1% to about 30% (w/w) of a phospholipid based on the weight of the oil base, about 1.6% to about 40% (w/w) of a non-proton-providing surfactant based on the weight of the oil base, and the balance of the composition being water.

The term "effective amount" as used herein, means that amount of PGE₁ to be included in an emulsion composition, which provides a therapeutically effective amount of PGE₁ to a subject when the emulsion composition is administered to the subject.

As used herein, the term "therapeutically effective amount" refers to that amount of PGE₁ that elicits a biological or medicinal response in a tissue system of a subject, or in a subject, that is being sought by a researcher, veterinarian, medical doctor or other clinician. The biological or medicinal response includes a clinically observable beneficial effect resulting from the treatment of a disease or disorder related to PGE₁. In one embodiment of the invention, a therapeutically effective amount of PGE₁ abolishes an existing disease or disorder related to PGE₁ when administered to a subject. In another embodiment of the invention, a therapeutically effective amount of PGE₁ reduces a disease or disorder related to PGE₁ before or after the occurrence of the disease or disorder to a degree that is less than about 10%, 20%, 30%, 40%, 50%, 60%, 70%, 80%, or 90% or more of what would have been observed when the subject has not received a therapeutically effective amount of PGE₁.

One skilled in the art will recognize that the "effective amount" of PGE₁ to be included in an emulsion composition may vary depending upon factors, such as the presence of other components in the emulsion composition, the dose range of the emulsion composition, the mode of administration of the emulsion composition, the characteristics of the particular PGE₁ used, etc. Standard procedures can be performed to evaluate the effect of the administration of PGE₁ to a subject, thus allowing a skilled artisan to determine the effective amount of PGE₁ to be included in an emulsion composition.

In the invention, the effective amount of PGE₁ may be, for example, about 0.1 microgram (µg) to about 100 µg, about 0.5 µg to about 50 µg, or about 1 µg to about 10 µg, per 1 milliliter (ml) of the PGE₁ emulsion composition. In one embodiment of the invention, the effective amount of PGE₁ is about 5 µg/ml (w/v) or about 0.0005% (w/w) based on the volume or the weight of the emulsion composition, respectively. However, the effective amount of PGE₁ that may be included in a PGE₁ emulsion composition is not limited to the exemplified amounts.

As used herein, the term "subject" refers to an animal, preferably a mammal, who has been the object of treatment, observation or study. Examples of a subject may be a human, a livestock animal (beef and dairy cattle, sheep, poultry, swine, etc.), or a companion animal (dogs, cats, horses, etc).

As used herein, the term "prostaglandin E₁ (PGE₁) " includes a compound of formula (I):

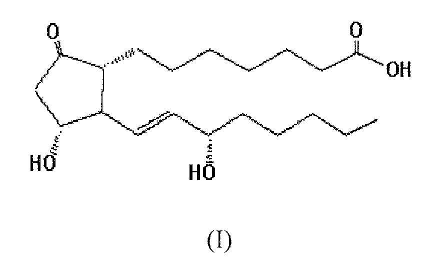

According to the invention, any pharmaceutically acceptable oil may be used as an oil base, including animal lipids, vegetable oils and synthesized oils, for example soybean oil, cottonseed oil, rapeseed oil, sesame oil, corn oil, peanut oil, sunflower oil, fish oil, and any triglycerides. "Triglyceride" as used herein includes a class of naturally occurring or synthetic lipids or esters in which each of the three hydroxyl groups of a glycerol is modified by a fatty acid. The fatty acids may be of the same kind or different kinds. The fatty acids may be saturated or unsaturated. The chain length of each fatty acid may be of varying lengths, for example about 6 to 26 carbon atoms. In an embodiment of the invention, the oil base is a medium chain triglyceride (MCT) oil. The term "medium chain triglyceride (MCT)" as used herein refers to a triglyceride containing one or more fatty acids each having a medium-size chain length of about 6 to about 14 carbon atoms or mixtures thereof. In one example of the invention, the MCT is a mixture of triglycerides, mainly containing not less than about 95% of saturated fatty acids with 8 and 10 carbon atoms, such as caprylic acid and capric acid. Particularly, the MCT is a mixture of triglycerides mainly containing not less than about 95% (w/w) of caprylic acid and capric acid, such as LIPOID MCT^{®} from Lipoid GmBH, Germany or Panacet 810^{®} (JPE) from NOF Corp., Japan. In another example of the invention, the MCT is a mixture of esters of saturated coconut and palm kernel oil derivatives of caprylic and capric fatty acids and glycerin or propylene glycol, such as Miglyol^{®} natural oils from Condea Chemie GmbH of Witten, Germany.

According to the invention, a phospholipid with a high purity as an emulsifier is added to the composition that may be any lipid molecule containing phosphorus. As used herein, "phospholipid" includes a class of molecules composed of a glycerol or a sphingosine backbone, and one or two fatty acid chains and a negatively charged phosphate group attached to the carbons on the backbone, wherein the negatively charged phosphate group is usually attached to a nitrogen-containing head group such as choline or inositol. A "phospholipid with a high purity" as used herein can be any phospholipid preparation that contains about 95% (w/w) or more of a pure phospholipid in the preparation, or any phospholipid preparation that has a purity of 95% (w/w) or more. While phospholipid preparations are commonly used in conventional PGE₁ emulsion compositions, such preparations have a lower purity, on the order of 70% (w/w). According to this invention, the phospholipid used herein is a phospholipid with a high purity. Examples of phospholipids that may be used in the present invention include, but are not limited to, phosphatidyl phospholipids, glycerophospholipids and sphingomyelins. Examples of phosphatidyl phospholipids include a phosphatidylcholine selected from the group consisting of hydrogenated egg phosphatidylcholine (HEPC), hydrogenated soy phosphatidylcholine (HSPC), dipalmitoyl phosphatidylcholine (DPPC) and distearoyl phosphatidylcholine (DSPC), diarachidoyl phosphatidylcholine, dimyristoyl phosphatidylcholine (DMPC), egg phosphatidylcholine (EPC) and soy phosphatidylcholine (SPC), oleoyl palmitoyl phosphatidylcholine, dioleoyl phosphatidylcholine (DOPC), dipetroselinoyl phosphatidylcholine, palmitoylelaidoyl phosphatidylcholine, palmitoyloleoyl phosphatidylcholine, dilauroyl phosphatidylcholine (DLPC), diundecanoyl phosphatidylcholine, didecanoyl phosphatidylcholine, and dinonanoyl phosphatidylcholine.

According to embodiments of the invention, a non-proton-providing surfactant is used as an auxiliary emulsifier in the composition. As used herein, the term "non-proton-providing surfactant" refers to an interface-active molecule or a surface active agent, which does not provide any proton or which provides not more than one mole proton per one hundred moles of the interface-active molecule or the surface active agent (one mole % proton) while the pH of an aqueous composition containing the interface-active molecule or the surface active agent is about 4 to about 8. In accordance with embodiments of the invention, the non-proton-providing surfactant may be a non-ionic surfactant, a cationic surfactant or a zwitterionic (also known as "amphoteric") surfactant. A surfactant is usually an organic compound that are amphiphilic, containing both a hydrophobic group ("tail") and a hydrophilic group ("head"). A nonionic surfactant has no formally charged groups in its head. A cationic surfactant carries a net positive charge in its head. A zwitterion surfactant is electrically neutral but carries formal positive and negative charges on different atoms. Examples of the non-proton-providing surfactant include a poloxamer such as polyalkylene glycols under the trade name of Pluronic® (for example, polyethylene glycols having an average molecular weight of about 1,000 to about 20,000 daltons, preferably about 4,000 daltons to about 6,000 daltons), polyalkylene copolymers (for example, polyoxyethylene-polyoxypropylene copolymers having an average molecular weight of about 1,000 daltons to about 20,000 daltons, preferably about 6,000 daltons to about 10,000 daltons); a castor oil-polyoxyalkylene derivative such as those available under the trade name Cremophor®, for example, polyoxyl 35 castor oil (Cremophor® EL), polyoxyl 40 hydrogenated castor oil and polyoxyl 60 hydrogenated castor oil; a polysorbate (for example, polyoxyethylene sorbitan monolaurate (polysorbate 20), polyoxyethylene sorbitan monopalmitate (polysorbate 40), polyoxyethylene sorbitan monostearate (polysorbate 60), polyoxyethylene sorbitan tristearate (polysorbate 65), polyoxyethylene sorbitan monooleate (polysorbate 80), such as those available under the trademark Tween®; or polyoxyethylene (15) esters of 12-hydroxysteric acid (polyethylene glycol 660 12-hydroxystearate) (Solutol® HS15), or a derivative of vitamin E such as d-alpha-tocopheryl polyethylene glycol 1000 succinate (TPGS), or a cationic surfactant such as dodecyltrimethylammonium bromide, or zwitterionic surfactants such as 3-(N,N-dimethyl palmitylammonio)-propanesulfonate. In particular embodiments of the invention, the non-proton-providing surfactant is selected from the group consisting of polyoxyethylene sorbitan monooleate (polysorbate 80), polyoxyl 35 castor oil (Cremophor® EL), d-alpha-tocopheryl polyethylene glycol 1000 succinate (TPGS), and polyoxyethylene (15) esters of 12-hydroxysterie acid (polyethylene glycol 660 12-hydroxystearate) (Solutol® HS 15). In another example of the invention, the non-proton-providing surfactant is polyethylene glycol derived phopholipid, containing a polyethylene glycol having a molecular weight of about 500 to about 10,000 daltons, such as 1,2-distearoly-*sn*-glycero-3-phosphoethanolamine-N-[methoxy(polyethylene glycol)-2000] (DSPE-mPEG2000).

Normally, an excessive amount of a surfactant or emulsifier will lead to the formation of micelles, aggregates of surfactant molecules dispersed in a liquid colloid. The formation of micelles in an emulsion composition comprising PGE₁ will increase the exposure of PGE₁ to water, and then initiate PGE₁ degradation, and therefore, should be avoided or minimized. Therefore, in the present invention, the amount of the non-proton-providing surfactant in the emulsion composition should be more than about 1.6% but not more than about 40% (w/w), such as about 1.6% to about 40% (w/w), preferably about 2% to about 15% based on the weight of the oil base.

In addition, the emulsion composition may also include a suitable amount of isotonifying agent for isotonification or stabilization of the emulsion. In an example of the invention, an isotonifying agent, such as glycerol, a saccharide or a salt in a proportion of about 0.05% to about 10% (w/w) may be added to the composition. The emulsion composition may have other additives, not limited by the components herein described.

In one particular example of the invention, the emulsion composition comprises about 0.0005% (w/w) of PGE₁ based on the weight of the emulsion composition, about 15% (w/w) of MCT oil as an oil base based on the weight of the emulsion composition, about 18% (w/w) of EPC with a high purity based on the weight of the oil base, about 2.4% (w/w) of polysorbate 80 based on the weight of the oil base, and the balance of the emulsion composition being water. In another particular example of the invention, the emulsion composition comprises about 0.0005% (w/w) of PGE₁ based on the weight of the emulsion composition, about 10% (w/w) of MCT oil as an oil base based on the weight of the emulsion composition, about 18% (w/w) of EPC with a high purity based on the weight of the oil base, about 2.4% (w/w) of DSPE-mPEG2000 based on the weight of the oil base, and the balance of the emulsion composition being water.

Another general aspect of the present invention relates to a method of improving the stability of PGE₁ in an emulsion composition. The method comprises mixing together an effective amount of PGE₁, a pharmaceutically acceptable oil, and about 1.6% to about 40% (w/w) of a non-proton-providing surfactant based on the weight of the pharmaceutically acceptable oil. Additional components may also be added and admixed in the emulsion composition, such as phospholipid with a high purity, water, stabilizers and isotonifying agents.

In an embodiment of the present invention, the emulsion composition was prepared by the method comprising mixing together an effective amount of PGE₁, about 1% to about 30% (w/w) of a pharmaceutically acceptable oil based on the weight of the emulsion composition, about 1% to about 30% (w/w) of a phospholipid with a high purity based on the weight of the oil base, about 1.6% to about 40% (w/w) of a non-proton-providing surfactant based on the weight of the oil base, and an appropriate amount of water.

The emulsion composition according to embodiments of this invention can be prepared by any standard method commonly used in the art in view of the present disclosure. In one embodiment of the invention, PGE₁, a phospholipid with a high purity, and a non-proton-providing surfactant in the predetermined amounts, as well as one or more additives if desired, are mixed with a required amount of an oil base to form a solution. An appropriate amount of water is added to the solution. The mixture is emulsified by means of a mechanical homogenizer such as a pressure-jet type homogenizer or an ultrasonicator to obtain a homogenized, extremely fine fat emulsion containing PGE₁.

Embodiments of the present invention further relate to a method of treating a disease or disorder related to PGE₁ using an emulsion composition according to embodiments of this invention. The method comprises administering to a subject an emulsion composition according to any of the embodiments of this invention in a therapeutically effective amount to treat the PGE₁-related disease or disorder. The therapeutically effective amount of the emulsion composition is to abolish, interdict, prevent, delay, palliate, or alleviate the disease or disorder in the subject.

The emulsion composition according to embodiments of this invention may be administered to a subject for the purposes for which prostaglandins are generally administered. The emulsion composition may be administered by injection or infusion into a vein (intravenous, IV), a muscle (intramuscular, 1M), or under the skin (subcutaneous, SC). In addition, transdermal, nasal (inhalant) or oral administration may also be used in the invention. The emulsion composition may also be administered by a catheter or port temporarily or permanently inserted into a central vein, body cavity, bladder or pelvis. For example without limitation, the emulsion composition according to embodiments of this invention may be administered to a subject by sustained intravenous infusion at the rate of about 0.02 ng to about 5 ng of PGE₁ and other therapeutic agents per kg of body weight per minute. Furthermore, in another example, the emulsion composition according to embodiments of this invention may be administered to a subject by injection.

The concomitant administration of PGE₁ in the composition in embodiments of the present invention may involve concurrent (i.e. at the same time), prior, or subsequent administration of the other therapeutic agent(s) with respect to the administration of an emulsion composition of the present invention. A person of ordinary skill in the art would have no difficulty determining the appropriate timing, sequence and dosages of administration for particular drugs and compounds of the present invention based on the disorder or disease and condition of the subject being treated, in view of the present disclosure.

The invention will now be described in further detail with reference to the following specific, non-limiting examples.

### Example 1

### Preparation of an Emulsion Composition TLC01 Comprising PGE₁ and Polysorbate 80

An emulsion composition comprising PGE₁ of formula (I) and a non-proton-providing surfactant, polysorbate 80, was prepared in this Example. Methods similar to that of this Example may be used to prepare an emulsion composition comprising any other PGE₁, and any other non-proton-providing surfactant.

The PGE₁ of formula (I) and other chemicals were purchased from Sigma, USA. Polysorbate 80 was purchased from Sigma, USA, NOF corp., Japan, or Imperial Chemical Industries PLC, London, United Kingdom. The MCT oil (a mixture of triglycerides mainly containing not less than about 95% of caprylic acid and capric acid), such as LIPOID MCT^{®} purchased from Lipoid GmbH, Germany or Panacet 810® from NOF Corp., Japan. Egg phosphatidylcholine (EPC) (purity: 95~100% and purity: 80 ~ 85%) were purchased from Avanti, USA, NOF Corp., Japan, or Lipoid GmbH, Germany.

A composition containing 0.9 g of EPC (purity: 95~100%), 500 µg of PGE₁, 5 g of purified MCT oil, 2.21 g of glycerol and 0.12 g of polysorbate 80 were then mixed with 91.77 g of distilled water for injection (WFI). The mixture was homogenized with an ultrasonicator (Sonicator 3000 by Misonix, Inc., Farmingdale, NY., USA). A homogenized, finely dispersed fat emulsion containing PGE₁ was obtained. The emulsion particle size was determined by a laser particle analyzer. The droplets of the emulsion had a size less than about 0.5 µm.

### Example 2

### Preparation of Emulsion Compositions Comprising PGE₁ and Other Non-Proton-Providing Surfactants

Using methods similar to that described in Example 1, different emulsion compositions comprising PGE₁ and different non-proton-providing surfactants were prepared.

Non-proton-providing surfactants were obtained from various sources. Polyethylene glycol-15-hydroxystearate (Solutol^{®} HS15) and polyoxyl 35 castor oil (Cremophor® EL) were obtained from BASF, Germany. D-alpha-tocopheryl polyethylene glycol 1000 succinate (TPGS) was obtained from Eastman, USA. 3-(N,N-dimethylpalmitylammonio)propanesulfonate and dodecyltrimethylammonium bromide were obtained from Sigma, USA. DSPE-mPEG2000 was purchased from Avanti, USA, or NOF Corp., Japan.

The components and their amounts in various emulsion compositions are listed in Table I.

**Table I Components and Their Amounts of the Emulsion Compositions**

| Formula | Oil Base (g) | Emulsifier (g) | Auxiliary emulsifier (g) | PGE₁ (mg) | Glycerol (g) | Water (g) |
|---|---|---|---|---|---|---|
| Liple^{®} | 10 (Soybean oil) | 1.8 (EPC (purity: 95~100%)) | 0.24 (Oleic acid) (Oleic acid) | 0.5 | 2.21 | 85.75 |
| A1 | 5 (MCT) | 0.9 (EPC (purity: 95~100%)) | 0.12 (Oleic acid) | 0.5 | 2.21 | 91.77 |
| A2 | 5 (MCT) | 0.9 (EPC (purity: 95~100%)) | None one | 0.5 | 2.21 | 91.89 |
| TLC01 | 5 (MCT) | 0.9 (EPC (purity: 95~100%)) | 0.12 (Polysorbate 80) | 0.5 | 2.21 | 91.77 |
| TLC02 | 5 (MCT) | 0.9 (EPC (purity: 95~100%)) | 0.6 (Solutol® HS15) | 0.5 | 2.21 | 91.29 |
| TLC03 | 5 (MCT) | 0.9 (EPC (purity: 95~100%)) | 2.0 (Cremophor® EL) | 0.5 | 2.21 | 89.5 |
| TLC04 | 5 (MCT) | 0.9 (EPC (purity: 95~100%)) | 0.12 (TPGS) | 0.5 | 2.21 | 91.77 |
| TLC05 | 5 (MCT) | 0.9 (EPC (purity: 95~100%)) | 0.08 (3-(N,N-Dimethyl palmitylammonio) propanesulfonate) | 0.5 | 2.21 | 91.42 |
| TLC06 | 5 (MCT) | 0.9 (EPC (purity: 95~100%)) | 0.08 (Dodecyltrimethyl ammonium bromide) | 0.5 | 2.21 | 91.42 |
| TLC07 | 10 (MCT) | 1.8 (EPC (purity: 95~100%)) | 0.24 (Polysorbate 80) | 0.5 | 2.21 | 85.75 |
| TLC08 | 15 (MCT) | 2.7 (EPC (purity: 95~100%)) | 0.36 (Polysorbate 80) | 0.5 | 2.21 | 79.73 |
| TLC09 | 20 (MCT) | 3.6 (EPC (purity: 95~100%)) | 0.48 (polysorbate 80) | 0.5 | 2.21 | 73.71 |
| TLC10 | 30 (MCT) | 5.4 (EPC (purity: 95~100%)) | 0.72 (Polysorbate 80) | 0.5 | 2.21 | 61.67 |
| TLC11 | 10 (MCT) | 1.8 (EPC (purity: 95~100%)) | 0.24 (DSPE-mPEG2000) | 0.5 | 2.21 | 85.75 |
| A3 | 10 (MCT) 80~85%)) | 1.8 (EPC (purity: | 0.24 (DSPE-mPEG2000) | 0.5 | 2.21 | 85.75 |

### Example 3

### Determination of Shelf Stability of PGE₁ in the Emulsion Compositions

Shelf stability of PGE₁ in an array of emulsion compositions was determined in this example. Methods similar to that of this Example may be used to determine shelf stability of any PGE₁ in any emulsion composition comprising PGE₁.

A commercially available product Liple^{®} (Mitsubishi Pharma Corp., Tokyo, Japan) was used as a reference in comparison with the emulsion compositions according to embodiments of the invention. The components and their amounts in various emulsion compositions for comparison are listed in Table 1.

The stability of PGE₁ in the emulsion compositions, which were stored in sealed clear ampoules and in the dark, was determined in terms of the percentages of PGE₁ retained after the incubation of the emulsion compositions at 40°C for one week or one month by HPLC analysis. The stability of PGE₁ in the emulsion compositions was also determined by the appearance (phase separation or not) of the emulsion compositions after the incubation period. The shelf stability test results are set forth in the following Table II.

**Table II Shelf Stability of the Emulsion Compositions**

| Formula | After 1 week incubation | | | | After 1 month incubation | | | |
|---|---|---|---|---|---|---|---|---|
| | PGE₁ retained (%) | | | Phase Separation | PGE₁ retained (%) | | | Phase Separation |
| Liple^{®} | 74.6 | ± | 1.3 | No | 21.3 | ± | 2.1 | No |
| A1 | 75.4 | ± | 0.6 | No | 32.0 | ± | 3.1 | No |
| A2 | 84.7 | ± | 4.1 | Yes | 70.6 | ± | 1.8 | Yes |
| TLC01 | 80.1 | ± | 2.8 | No | 50.6 | ± | 2.5 | No |
| TLC02 | 82.7 | ± | 1.1 | No | 53.3 | ± | 2.7 | No |
| TLC03 | 84.2 | ± | 1.1 | No | 55.5 | ± | 0.3 | No |
| TLC04 | 82.5 | ± | 1.9 | No | 48.6 | ± | 2.6 | No |
| TLC05 | 88.6 | ± | 1.2 | No | 56.6 | ± | 1.4 | No |
| TLC06 | 76.5 | ± | 1.6 | No | 31.1 | ± | 0.3 | No |
| TLC07 | 82.1 | ± | 0.6 | No | 53.0 | ± | 3.6 | No |
| TLC08 | 85.9 | ± | 1.8 | No | ---- | | ---- | ---- |
| TLC09 | 84.2 | ± | 2.1 | No | ---- | | ---- | ---- |
| TLC10 | 87.1 | ± | 0.5 | No | ---- | | ---- | ---- |
| TLC11 | 81.7 | ± | 0.4 | No | ---- | | ---- | ---- |
| A3 | 59.1 | ± | 1.5 | No | | | | |

The results shown in Table Il demonstrate that in the emulsion compositions comprising oleic acid (a proton-providing surfactant), the amounts of PGE₁ remaining after one month were less than those in the emulsion compositions comprising a non-proton-providing surfactant according to embodiments of the invention.

During the storage of either one week or one month at 40°C, no phase separation was observed in the emulsion compositions according to the invention, including Formula TLC01 comprising polysorbate 80, which was prepared in accordance with Example 1, as well as Formula TLC01 and TLC03 comprising Solutol^{®} HS 15 and Cremophor^{®} EL, respectively, which were prepared in accordance with Example 2. It was evident from the results of Table II that a non-proton-providing surfactant, instead of oleic acid (a proton-providing surfactant), when used as an auxiliary emulsifier in emulsion compositions, at a concentration of at least about 1.6% (w/w) based on the weight of the oil base in the emulsion compositions, resulted in improved stability of PGE₁ in the emulsion compositions.

The results shown in Table II demonstrated that in the composition of formula A3 comprising a EPC (purity: 80 ~ 85%), the amounts of PGE₁ remaining after one week was less than those in the composition of formula TLC11 comprising a high purity of EPC (purity: 95 ~ 100%) according to the invention.

It will be appreciated by those skilled in the art that changes could be made to the embodiments described above without departing from the broad inventive concept thereof. It is understood, therefore, that this invention is not limited to the particular embodiments disclosed, but it is intended to cover modifications within the spirit and scope of the present invention as defined by the appended claims.

## Claims

1. An emulsion composition comprising:
an effective amount of PGE₁, about 1% to about 30% (w/w) of a pharmaceutically acceptable oil as an oil base based on the weight of the emulsion composition, about 1% to about 30% (w/w) of a phospholipid with a high purity based on the weight of the oil base, about 1.6% to about 40% (w/w) of a non-proton-providing surfactant based on the weight of the oil base, and the balance of the emulsion composition being water.

2. The emulsion composition according to claim 1, wherein the non-proton-providing surfactant does not provide proton or provide not more than one mole % proton while the pH of an aqueous composition containing the non-proton-providing surfactant varies between about 4 and about 8.

3. The emulsion composition according to claim 1, wherein the non-proton-providing surfactant is a non-ionic surfactant, a cationic surfactant or a zwitterionic surfactant.

4. The emulsion composition according to claim 1, wherein the non-proton-providing surfactant is a non-ionic surfactant.

5. The emulsion composition according to claim 1, wherein the non-proton-providing surfactant is a polysorbate.

6. The emulsion composition according to claim 5, wherein the non-proton-providing surfactant is polysorbate 80.

7. The emulsion composition according to claim 1, wherein the non-proton-providing surfactant is a castor oil-polyoxyalkylene derivative.

8. The emulsion composition according to claim 7, wherein the non-proton-providing surfactant is polyoxyl 35 castor oil.

9. The emulsion composition according to claim 1, wherein the non-proton-providing surfactant is polyethylene glycol 660 12-hydroxystearate.

10. The emulsion composition according to claim 1, wherein the non-proton-providing surfactant is a polyethylene glycol derived phopholipid, containing polyethylene glycol having a molecular weight of about 500 to about 10,000 daltons.

11. The emulsion composition according to claim 10, wherein the non-proton-providing surfactant is 1,2-distearoly-*sn*-glycero-3-phosphoethanolamine-N-[methoxy(polyethylene glycol)-2000] (DSPE-mPEG2000).

12. The emulsion composition according to claim 1, wherein the high purity is more than about 95% (w/w).

13. The emulsion composition according to claim 1, wherein the phospholipid is phosphatidylcholine selected from the group consisting of hydrogenated egg phosphatidylcholine (HEPC), hydrogenated soy phosphatidylcholine (HSPC), dipalmitoyl phosphatidylcholine (DPPC), distearyloyl phosphatidylcholine (DSPC), diarachidoyl phosphatidylcholine, dimyristoyl phosphatidylcholine (DMPC), egg phosphatidylcholine (EPC), soy phosphatidylcholine (SPC), oleoyl palmitoyl phosphatidylcholine, dioleoyl phosphatidylcholine (DOPC), dipetroselinoyl phosphatidylcholine, palmitoylelaidoyl phosphatidylcholine, palmitoyloleoyl phosphatidylcholine, dilauroyl phosphatidylcholine (DLPC), diundecanoyl phosphatidylcholine, didecanoyl phosphatidylcholine, and dinonanoyl phosphatidylcholine.

14. The emulsion composition according to claim 1, wherein the phospholipid is egg phosphatidyl choline (EPC) with a purity more than about 95% (w/w).

15. The emulsion composition according to claim 1, wherein the oil base comprises a triglyceride.

16. The emulsion composition according to claim 15, wherein the oil base comprises a medium chain triglyceride (MCT) oil.

17. The emulsion composition according to claim 16, wherein the MCT is a mixture of triglycerides containing not less than about 95% (w/w) of caprylic acid and capric acid.

18. The emulsion composition according to claim 1, wherein the non-proton-providing surfactant is present in an amount of about 2% to about 15% (w/w) based on the weight of the oil base.

19. An emulsion composition comprising:
about 0.0005% (w/w) of PGE₁ based on the weight of the emulsion composition, about 15% (w/w) of MCT oil as an oil base based on the weight of the emulsion composition, about 18% (w/w) of EPC with a purity more than about 95% (w/w) based on the weight of the oil base, about 2.4% (w/w) of polysorbate 80 based on the weight of the oil base, and the balance of the emulsion composition being water.

20. An emulsion composition comprising:
about 0.0005% (w/w) of PGE₁ based on the weight of the emulsion composition, about 10% (w/w) of MCT oil as an oil base based on the weight of the emulsion composition, about 18% (w/w) of EPC with a purity more than about 95% (w/w) based on the weight of the oil base, about 2.4% (w/w) of DSPE-mPEG2000 based on the weight of the oil base, and the balance of the emulsion composition being water.

21. A method of improving the stability of PGE₁ in an emulsion composition, comprising mixing together an effective amount of PGE₁, a pharmaceutically acceptable oil, an emulsifier, and about 1.6% to about 40% (w/w) of a non-proton-providing surfactant based on the weight of the pharmaceutically acceptable oil.

22. A method of treating a subject having or being susceptible to a disease or disorder treatable by PGE₁, comprising administering to the subject a therapeutically effective amount of an emulsion composition according to claim 1.

23. The method according to claim 22, comprising administering the emulsion composition of claim 1 via infusion or injection.
